(19) Europäisches Patentamt
European Patent Office
Office européen des brevets

(11) EP 1 792 609 A1

(12) # EUROPÄISCHE PATENTANMELDUNG

(43) Veröffentlichungstag:
**06.06.2007 Patentblatt 2007/23**

(21) Anmeldenummer: **06023972.0**

(22) Anmeldetag: **18.11.2006**

(51) Int Cl.:
***A61K 8/894*** *(2006.01)* ***A61K 8/898*** *(2006.01)*
***A61Q 1/02*** *(2006.01)* ***A61Q 15/00*** *(2006.01)*
***A61Q 17/04*** *(2006.01)* ***A61Q 19/10*** *(2006.01)*
***C08G 77/10*** *(2006.01)* ***C08G 77/12*** *(2006.01)*
***C08G 77/14*** *(2006.01)* ***C08G 77/26*** *(2006.01)*
***C08G 77/38*** *(2006.01)* ***C08G 77/388*** *(2006.01)*
***C08G 77/46*** *(2006.01)* ***C09D 7/12*** *(2006.01)*
***C11D 1/82*** *(2006.01)* ***C11D 3/37*** *(2006.01)*

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **03.12.2005 DE 102005057857**

(71) Anmelder: **Goldschmidt GmbH**
**45127 Essen (DE)**

(72) Erfinder:
- **Grüning, Burghard, Dr.**
  **45134 Essen (DE)**
- **Knott, Wilfried, Dr.**
  **45355 Essen (DE)**
- **Leidreiter, Holger, Dr.**
  **45529 Hattingen (DE)**
- **Meyer, Jürgen, Dr.**
  **48143 Münster (DE)**

(54) **Organomodifizierte Polysiloxane mit Blockcharakter, Verfahren zu deren Herstellung und deren Verwendung**

(57) Gegenstand der Erfindung ist ein Verfahren zur Herstellung von organomodifizierten Siloxanen mit domänenartiger Verteilung erhalten durch teilweise oder vollständige Umsetzung von
A) Wasserstoffsiloxanen mit einem Verteilungsgrad (persistency ratio) (η) der Komponenten [A] und [B] im Copolymeren [AB]

$$\eta = \frac{[A]\,[B]}{[AB]}$$

von $\eta > 1$, vorzugsweise $> 1{,}1$, insbesondere $\geq 1{,}2$, mit
B) olefinisch und/oder acetylenisch ungesättigten Verbindungen, die daraus resultierenden Verbindungen und ihre Verwendung.



Printed by Jouve, 75001 PARIS (FR)

## Beschreibung

Die Erfindung betrifft durch Hydrosilylierung hergestellte organomodifizierte Polysiloxane mit Blockcharakter und deren Verwendung zur Herstellung kosmetischer und pharmazeutischer Formulierungen.

Ein großer Teil kosmetischer und pharmazeutischer Formulierungen besteht aus Emulsionen. Die Mehrzahl dieser Emulsionen ist üblicherweise vom Typ Öl-in-Wasser, d. h. die Ölphase ("disperse Phase") ist in Form kleiner Tröpfchen in der Wasserphase ("kohärente Phase") feinst verteilt. Weiterhin sind auch Wasser-in-Öl-Emulsionen bekannt, die sich dadurch auszeichnen, dass Wassertropfen in einer kontinuierlichen Ölphase dispergiert vorliegen. Diese Wasser-in-Öl-Emulsionen zeigen eine Reihe anwendungstechnischer Vorteile. So vermögen sie beispielsweise einen occlusiven Film auf der Hautoberfläche zu bilden, der ein Austrocknen der Haut verhindert. Gleichzeitig sind Wasser-in-Öl-Emulsionen bekannt für ihre ausgezeichnete Wasserfestigkeit.

Kosmetische Emulsionen enthalten, unabhängig vom Emulsionstyp, aus Gründen des Hautgefühls im Durchschnitt 20 bis 30 % Ölphase. Für den Fall von Wasser-in-Öl-Emulsionen bedeutet dies, dass aufgrund des hohen internen Phasengehalts eine extrem hohe Stabilisierung der Wassertropfen gegen Koaleszenz erforderlich ist.

Es ist bekannt, dass diese Stabilisierung zum Beispiel auf herausragende Art und Weise durch kammartig aufgebaute Alkylpolyether-Siloxane erfolgen kann, wie sie in EP-A-0 176 884 beschrieben sind.

Weit verbreitet ist auch der Einsatz endständig modifizierter Polyethersiloxane in kosmetischen Emulsionen. Diese Verbindungen zeichnen sich neben ihrer emulsionsstabilisierenden Wirkung durch ein samtig-seidiges Hautgefühl aus, dass sie kosmetischen Emulsionen verleihen.
Ein derartiger Öl-in-Wasser-Emulgator ist beispielsweise in EP-A-1 125 574 beschrieben.

Eine andere prominente Stoffklasse der organomodifizierten Siloxane für kosmetische Anwendungen beruht auf den reinen Alkylgruppen-modifizierten Siloxanen oder Siliconwachsen, die Anwendung finden z.B. bei der Spreitverbesserung von kosmetischen Ölen oder aber auch als netzende Dispergieradditive zur Herstellung farbverbesserter pigmenthaltiger Formulierungen.

Schließlich gehört auch die Verwendung von mit kationischen Organogruppen-modifizierten Siloxanen insbesondere zur Haarpflege, wie sie beispielsweise in der EP-B-0 294 642 beschrieben werden, zum Stand der Technik.

Die stetig wachsenden Ansprüche an kosmetische Formulierungen beispielsweise in Richtung einer größeren Formulierungsflexibilität oder hinsichtlich eleganterer sensorischer Profile kann mit den beschriebenen Siloxanderivaten aus dem Stand der Technik nicht weiter verbessert werden. Somit bestand ein Bedarf nach neuartigen Siloxanen, mit deren Hilfe weiter verbesserte Formulierungseigenschaften gezielt eingestellt werden können.

Nach einem bislang noch nicht veröffentlichten Vorschlag (Aktenzeichen 10 2005 001 039.3), auf den auch im Hinblick auf die vorliegende Erfindung vollumfänglich Bezug genommen wird, wird ein Verfahren zur gezielten Herstellung von domänenartig mit SiH-Funktionen durchsetzten Polydimethylsiloxan-Poly(methylhydrogen)siloxan-Copolymeren beschrieben.

Hierbei werden Gemische bestehend aus Methylhydrogenpolysiloxan, Hexamethyldisiloxan und Siloxancyclen an einem makrovernetzten, Sulfonsäuregruppen enthaltenden Kationenaustauscherharz bei einer Temperatur von 10 °C bis 120 °C in Kontakt gebracht und die erhaltenen äquilibrierten Organosiloxane isoliert. Das hierbei eingesetzte Kationenaustauscherharz ist dadurch charakterisiert, dass dessen Produkt P aus seiner spezifischen Oberfläche und aus dessen mittleren Porendurchmesser P < 2,2 x 10-3 $m^3$/kg, insbesondere < 1,5 x 10-3 $m^3$/kg, besonders bevorzugt < 1 x 10-3 $m^3$/kg und die spezifische Oberfläche A < 50 $m^2$/g, insbesondere A < 35 $m^2$/g und besonders bevorzugt A < 25 $m^2$/g beträgt.

Die derartig gekennzeichnete Ionenaustauscherphase gewährleistet je nach Auslegung die partielle oder auch überwiegende Präsenz von Methylhydrogensiloxy-Domänen im erhaltenen Polydimethylsiloxan-Poly(methylhydrogen)siloxan-Copolymeren.

Die Definition für Domäne wird inhaltlich in Übereinstimmung mit der Definition "persistence ratio" in der Publikation von P. Cancouet et al "Functional Polysiloxanes, I. Microstructure of Poly(hydrogenmethylsiloxane-co-dimethylsiloxane)s Obtained by Cationic Copolymerization", J. of Polymer Science, Par A: Polymer Chemistry, Vol 38, 826-836 (2000) gebraucht. Danach ist persistency ratio (η) eine Maßzahl für den Verteilungsgrad der Komponenten [A] und [B] im Copolymeren [AB]:

$$\eta = \frac{\bar{L}_{exp}}{\bar{L}_{st}} = \frac{[A]\,[B]}{[AB]}$$

Danach kennzeichnet die persistency ratio η das Verhältnis von experimentell bestimmter Blocklänge einer Monomersequenz in einem Copolymer zu der statistisch erwarteten Blocklänge bei vollkommener Verteilung.

Danach tendieren die Komponenten [A] und [B] bei η < 1 zu einer alternierenden, bei η = 1 zu einer regellösen und bei η > 1 zu einer domänenartigen Verteilung. Analytisch lässt sich die Kumulation von SiH-Funktionalitäten durch die Auswertung hochauflösender 29Si-NMR-Spektren belegen. Die erfindungsgemäß verwendeten Äquilibrierungsprodukte gemäß der Anmeldung mit dem Aktenzeichen: 10 2005 001 039.3 weisen η Werte > 1 auf.

Der Inhalt der obigen Publikation wird hiermit als Referenz eingeführt und gilt als Teil des Offenbarungsgehaltes der vorliegenden Erfindung.

Diese Produkte sind insbesondere deshalb bemerkenswert, da es mit den bisher bekannten Äquilibrierungskatalysatoren nicht möglich war, blockartig aufgebaute Äquilibrate, d.h. mit entlang der Siloxanstruktur lokal erhöhten Funktionalisierungsdichten (SiH-Cluster), gezielt und reproduzierbar herzustellen.

In einer zweiten Reaktionsstufe können die so hergestellten domänenartig aufgebauten Wasserstoffsiloxane mit Mehrfachbindungen aufweisenden Kohlenwasserstoffen und/oder ungesättigten Polyoxyalkylethern umgesetzt werden.

Diese zweite Stufe der Herstellung kann dabei in Anlehnung an die bekannten Hydrosilylierungsverfahren erfolgen, wie beispielsweise beschrieben in den Patentschriften US-3 234 252, US-4 047 958, US-3 427 271.

Allerdings zeigten erste Anwendungstests an so hergestellten blockartigen Polyethersiloxanen für technische Applikationen wie z.B. zur Stabilisierung von Polyurethanweichschäumen, dass die Performance dieser domänenartig strukturierten Polyethersiloxane im Vergleich zu konventionell hergestellten statistischen Polyethersiloxanen ganz erheblich schlechter war.

Umso überraschender wurde nun gefunden, dass die Umsetzungsprodukte aus den in der erster Stufe hergestellten domänenartigen strukturierten Wasserstoffsiloxanen mit Mehrfachbindungen aufweisenden Kohlenwasserstoffen und/oder ungesättigten Polyoxyalkylethern grenzflächaktive Produkte mit neuartigen, ausgezeichneten Anwendungseigenschaften in kosmetischen und pharmazeutischen Formulierungen ergeben.

Bei der Verwendung blockartig aufgebauter Alkylpolyethersiloxane als Emulgatoren für Wasser-in-Öl-Emulsionen zeigte sich völlig überraschend, dass eine ähnlich hohe emulsionsstabilisierende Wirkung erzielt werden kann wie durch den Einsatz statistisch-uniformer Alkylpolyethersiloxane.

Vorteilhaft am Einsatz der blockartig aufgebauten Polyethersiloxane ist darüber hinaus die niedrigere Emulsionsviskosität. Diese geringere Emulsionsviskosität bietet den Vorteil einer besseren Verteilbarkeit der Formulierung auf der Haut, eines verbesserten Einziehverhaltens sowie eines generell etwas leichteren Hautgefühls. Dabei scheinen die blockartig aufgebauten Alkylpolyethersiloxane generell ein ausgeprägteres samtig-seidiges Hautgefühl zu vermitteln als dies von den statistisch aufgebauten Alkylpolyethersiloxanen bekannt ist.

Überraschenderweise zeigte sich weiterhin, dass die blockartig modifizierten Alkylpolyethersiloxane insbesondere bei Verwendung hoher Anteile von Siliconölen (z.B. Cyclopentasiloxane, Dimethicone) in der Ölphase, ein ähnlich samtig-seidiges Hautgefühl zu vermitteln vermögen, wie dies bisher nur von endständig modifizierten Polyethersiloxanen bekannt war, die herkömmlicherweise als Emulgatoren zur Stabilisierung solcher Wasser-in-Silicon-Emulsionen eingesetzt werden. Gleichzeitig zeigen die domänenartig strukturierten Alkylpolyethersiloxane allerdings eine deutlich bessere Emulsionsstabilisierung als diese.

Ganz generell kann gesagt werden, dass das samtig-seidene Hautgefühl, das insbesondere Organosiloxane mit längeren unmodifizierten Siloxanketten auszeichnet, mit Hilfe dieser neuen domänenartig-strukturierten Organosiloxane wesentlich leichter mit den für den jeweiligen Anwendungszweck benötigten Funktionalitäten kombiniert werden kann als dies für die bisher erhältlichen statisch verteilten Organosiloxane möglich war.

So zeigten sowohl domänenartig aufgebaute Emulgatoren auf Basis von Alkylpolyethersiloxanen als auch Performanceverbesserer auf Basis von Alkylsiloxanen ein verbessertes Hautgefühl. Haarpflegeadditive auf Basis kationisch modifizierter domänenartiger Organosiloxane zeichneten sich durch einen geschmeidigeren Griff aus.

Ein Gegenstand der Erfindung ist daher ein Verfahren zur Herstellung von organomodifizierten Siloxanen mit domänenartiger Verteilung erhalten durch teilweise oder vollständige Umsetzung von

A) Wasserstoffsiloxanen mit einem Verteilungsgrad (persisteny ratio) (η) der Komponenten [A] und [B] im Copolymeren [AB]:

$$\eta = \frac{[A]\,[B]}{[AB]}$$

von η > 1, vorzugsweise > 1,1, insbesondere ≥ 1,2, mit

B) olefinisch und/oder acetylenisch ungesättigten Verbindungen.

Ein weiterer Gegenstand der Erfindung ist ein Verfahren zur Herstellung von organomodifizierten Siloxanen, welches dadurch gekennzeichet ist, dass man zur Herstellung der Methylhydrogensiloxane ein Gemisch aus Hexamethyldisiloxan, Poly(methyl)-wasserstoffsiloxan und Siloxancyclen als Ausgangsmaterial äquilibriert.

Ein weiterer Gegenstand der Erfindung ist ein Verfahren zur Herstellung von organomodifizierten Siloxanen, welches dadurch gekennzeichet ist, dass olefinisch ungesättigte Verbindungen der allgemeinen Formel (1) $CH_2=CHR-(CH_2)_n-A$ verwendet werden, worin A mindestens ein Rest ist, ausgesucht aus der Gruppe der

1. gegebenenfalls substituierten, gegebenenfalls funktionelle Gruppen enthaltende lineare und/oder cyclische Kohlenwasserstoffe,

2. statistisch oder blockweise aufgebauten Oxyalkylenketten-$(OAlk)_a$-R, worin

R H, ein gegebenenfalls substituierter und/oder gegebenenfalls Heteroatome enthaltender $C_{1-18}$-Alkylrest oder der Rest einer einbasischen und/oder mehrbasischen $C_{1-22}$-Carbonsäure welche gegebenenfalls Hydroxylgruppen enthalten kann, der Rest einer anorganischen Säure ausgesucht aus der Gruppe Schwefelsäure, Sulfonsäure, Phosphorsäure,

Oalk der Rest $-(EO)_b-(PO)_c-(BO)_d-(DO)_e-(SO)_f-$ mit
EO Ethylenoxidrest,
PO Propylenoxidrest,
BO Butylenoxydrest,
DO Dodecenyloxidrest,
SO gegebenefalls Alkyl-substituierter Styroloxidrest,
b,c,d,e,f $\geq 0$ sind, insbesondere b 0 bis 50, c 0 bis 50,
d,e,f gleich oder verschieden 0 bis 10 und die Summe b + c + d + e + f = a beträgt und
a 1 bis 50, vorzugsweise 1 bis 30, insbesondere 3 bis 20 ist,

und

3. Oxirangruppen enthaltende Reste der Formeln 3a bis 3d

3a 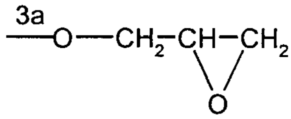

3b 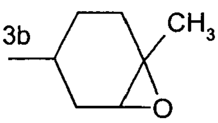

(und bei x = 0)

3c 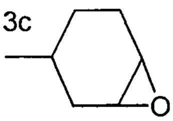

3d 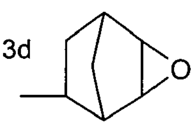

4. mindestens ein Amin-/Ammoniumrest, -MZ, $-[M-Z]^{g+}\ h{*}X^{j-}$,
worin

M ein zweiwertiger Rest ist, ausgewählt aus der Gruppe

4a 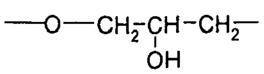 4b 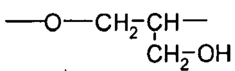 4c 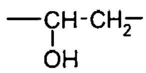 4d 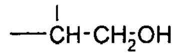

4e 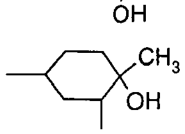 4f 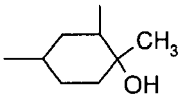 4g 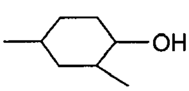 4h 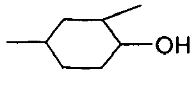

4j 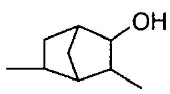 4k 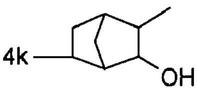

und Z einer der Reste

4m 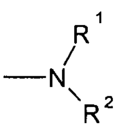 4n 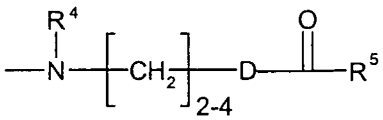 4o 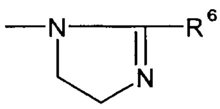

4p 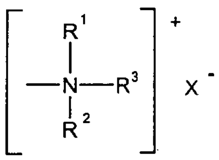 4q 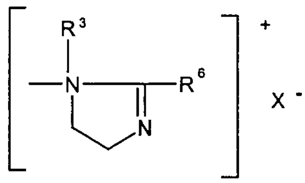 4r 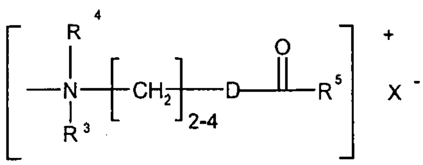

sein kann, worin

| | |
|---|---|
| $R^1$, $R^2$, $R^3$, $R^4$ | unabhängig voneinander H, $C_{1-22}$-Alkylreste die auch Hydroxylgruppen enthalten können, |
| $R^5$, $R^6$ | $C_{1-22}$-Alkylreste die auch Hydroxylgruppen enthalten können, |
| D | -O- oder -$NR^7$- mit $R^7$= Alkyl- oder Hydroxyalkylrest mit 1 bis 4 C-Atomen, |
| $X^-$ | ein organisches oder anorganisches Anion sein kann, |
| h*j | gleich dem Zahlenwert von g ist; |

5. Polyhydroxyorganylreste der allgemeinen Formel -$R^8$-PH,
wobei der Rest

$R^8$ als Spacer zwischen Siloxangerüst und Polyhydroxyorganylrest bzw. Zuckerrest fungiert und von aus dem Stand der Technik für polyhydroxyorganyl- bzw. zuckermodifizierte Siloxane bekannter Art ist,

PH ein Polyhydroxyorganylrest ist, der eine definierte Anzahl n von (C-OH)-Gruppen enthält, wobei n ≥ 2 ist, bevorzugt 5 bis 15 aus der Gruppe Mono-, Di-, Oligo- oder Polysaccharid, deren Glycoside oder entsprechende Derivate insbesondere Glucose, Maltose, Raffinose, Sorbit, Glucosamin, Glucopyranosylamin, Glucamin, N-Methylglucamin, Isomaltamin, Gluconsäure, Heptagluconsäure;

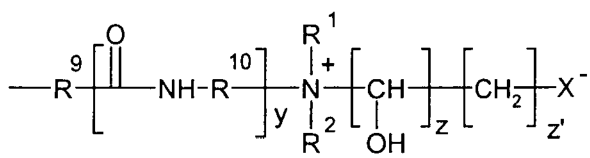

6. Betaingruppen,
worin

X -COO-, $SO_3^-$, $PO_4^{2-}$,
$R^9$ Alkylenreste mit bis zu 10 C-Atomen,
$R^{10}$ Alkylenreste mit bis zu 2 bis 6 C-Atomen sind,
y 0 oder 1,
Z 0 oder 1,
z' 1,2 oder 3,

und $R^1$, $R^2$ gleich oder verschieden die oben genannte Bedeutung haben, zusammen einen Imidazolinring bilden oder $-CH_2-CH_2-OH$, $-(CH_2)_z-X^-$ sind.

7. Guanidingruppen der Formeln (6a, 6b oder 6c)

$$R^{11} = -N-G \quad (6a)$$

$$R^{11} = -N-Q^+ A^- \quad (6b)$$

$$R^{11} = -(N)_x-S \quad (6c)$$

worin

G eine Guanidinogruppe mit der allgemeinen Formel ($6a^1$, $6a^2$)

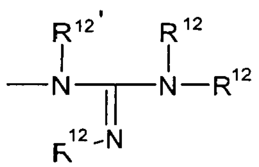
($6a^1$)

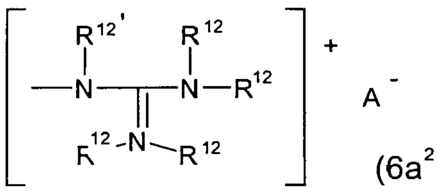
($6a^2$)

und/oder deren Salze oder Hydrate ist, in denen

| | |
|---|---|
| $R^{12}$ | unabhängig voneinander, Wasserstoff oder ein gegebenenfalls verzweigter, gegebenenfalls Doppelbindungen enthaltender Kohlenwasserstoffrest, oder |
| $R^{12'}$ $R^{12}$ | sein kann oder eine Alkylengruppe, die mit M über Kohlenstoff- oder Heteroatome verknüpft ist und so einen 5 bis 8-gliedrigen Ring bildet und |
| N | ein zwei- oder mehrwertiger Kohlenwasserstoffrest mit mindestens 4 Kohlenstoffatomen ist, der eine Hydroxylgruppe aufweist und der durch ein oder mehrere Sauerstoffatome oder Stickstoffatome oder quaternäre Ammoniumgruppen oder Ester oder Amidfunktionen unterbrochen sein kann, |
| $Q^+$ | ein Rest der Formel (6d) |

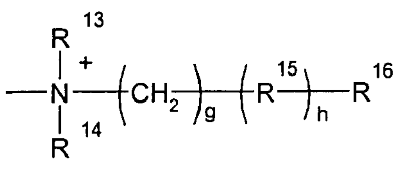
(6d)

ist,

$R^{13}$, $R^{14}$ Alkylreste mit 1 bis 4 Kohlenstoffatomen sind,

$R^{15}$ 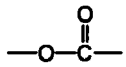 oder 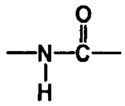

ist,

$R^{16}$ ein einwertiger Kohlenwasserstoffrest mit 1 bis 22 Kohlenstoffatomen sein kann,

g 0 bis 6

h 0 oder 1,

$A^-$ ein anorganisches oder organisches Anion, das von einer üblichen physiologisch verträglichen Säure HA herrührt,

S H, ein Polyalkylenoxid-Polyether der allgemeinen Formel $C_mH_{2m}O(C_2H_4O)_n(C_3H_6O)_oR^{17}$

worin

m 1 bis 6, insbesondere 3, 6,

n,o unabhängig voneinander 0 bis 100, insbesondere 0 bis 20 und der Polyether ein Molekulargewicht zwischen 100 und 6.000 g/mol aufweist und

$R^{17}$ H oder ein gegebenenfalls verzweigter, gegebenenfalls Doppelbindungen enthaltender aromatischer oder alicyclischer Kohlenwasserstoffrest mit 2 bis 30 C-Atomen, vorzugsweise 4 bis 22 C-Atomen, oder eine UV-absorbierende Gruppe, insbesondere Zimtsäure oder Methoxyzimtsäure ist.

Die aufgeführten Verbindungen werden nach, an sich bekannten Verfahren mit den SiH-Siloxanen verknüpft. Die Verbindungen sowie die Verfahren zur Herstellung der organomodifizierten Siloxane werden detailliert in den Patenten US-6,645,842, US-4,698,178 US-5,204,433 US-4,891,166 US-4,833,225, US-4,609,750 beschrieben. Der Inhalt dieser Patente wird hiermit als Referenz eingeführt und gilt als Teil des Offenbarungsgehaltes der vorliegenden Erfindung.

Ein weiterer Gegenstand der Erfindung ist die Verwendung der gemäß dem erfindungsgemäßen Verfahren erhaltenen domänenartig aufgebauten Organopolysiloxane zur Herstellung von kosmetischen Formulierungen. Diese zeigen hervorragende Anwendungseigenschaften.

Die erfindungsgemäßen blockartig aufgebauten Organosiloxane können dabei beispielsweise je nach Art, Anzahl und Verteilung der Substituenten als Emulgatoren oder Dispergieradditive, als Additive für ein verbessertes Hautgefühl oder einen verbesserten Griff, oder allgemein als Performanceverbesserer etwa zur besseren Verarbeitung anderer Inhaltsstoffe eingesetzt werden.

Ein weiterer Gegenstand der Erfindung ist der Einsatz der erfindungsgemäßen domänenartig aufgebauten Organosiloxane in kosmetischen Formulierungen zur Pflege und Reinigung von Haut und Haar, in Sonnenschutzprodukten, in Antitranspirantien/Deodorantien, sowie in pigmenthaltigen Formulierungen aus dem Bereich der dekorativen Kosmetik.

Ein weiterer Gegenstand der Erfindung ist die Nutzung der erfindungsgemäß hergestellten Verbindungen in pharmazeutischen Formulierungen.

Ein weiterer Gegenstand der Erfindung ist der Einsatz der erfindungsgemäßen domänenartig aufgebauten Organosiloxane in Mitteln zur Reinigung und Pflege harter Oberflächen, sowie zur Reinigung und Pflege von Textilien.

Darüber hinaus ist die Verwendung der erfindungsgemäß beanspruchten Verbindungen als Lackadditive in Form von z.B. Substratnetzmitteln, Gleit- und Verlaufsverbesserern, Entlüftern, Kratzfestigkeitsverbesserern u.a. Gegenstand der Erfindung.

Bevorzugt ist der Einsatz der erfindungsgemäßen grenzflächenaktiven Verbindungen in kosmetischen Formulierungen zur Pflege und Reinigung von Haut und Haar. Dabei kann es sich beispielsweise um Cremes oder Lotionen zur Hautpflege, Produkte auf tensidischer Basis zur Reinigung und Pfege von Haut und Haar, Sonnenschutzprodukte, pigmenthaltige Produkte aus dem Bereich der dekorativen Kosmetik (z.B. Make Up, Lippenstifte, Puder, Produkte für Lid/Wimperncolorierung), Produkte zur Konditionierung von Haar, Nagelpflegeprodukte oder um Antiperspirantien/Deodorantien handeln.

In diesen Formulierungen können die erfindungsgemäßen Verbindungen zusammen mit den für diese Anwendungsgebiete bekannten Rezepturbestandteilen wie Ölkomponenten, Co-Tensiden und Co-Emulgatoren, Konsistenzgebern, Verdickern, Wachsen, UV-Lichtschutzfiltern, Antioxidantien, Hydrotropen, Deodorant- und Antitransparantwirkstoffen, Wirkstoffen, Farbstoffen, Konservierungsmittel und Parfüms eingesetzt werden.

Als kosmetische Öle kommen insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen in Betracht.

Ebenso sind im erfindungsgemäßen Sinne die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Als Ölkomponenten geeignete Monoester sind z. B. die Methylester und Isopropylester von Fettsäuren mit 12 bis 22 C-Atomen wie z. B. Methyllaurat, Methylstearat, Methyloleat, Methylerucat, Isopropylpalmitat, Isopropylmyristat, Isopropylstearat, Isopropyloleat. Andere geeignete Monoester sind z. B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z. B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z. b. im Jojobaöl oder im Spermöl vorliegen.

Geeignete Dicarbonsäureester sind z. B. Di-n-butyl-adipat, Din-butyl-sebacat, Di-(2-ethylhexyl)-adipat, Di-(2-hexyldecyl)-succinat, Di-isotridecylazelat. Geeignete Diolester sind z. B. Ethylenglycoldioleat, Ethylenglycol-di-isotridecanoat, Propylenglycol-di-(2-ethylhexanoat), Butandiol-di-isostearat und Neopentylglycol-di-caprylat.

Weitere Fettsäureester, die eingesetzt werden können, sind die Veresterungsprodukte von Benzoesäure mit linearen oder verzweigten Fettsäuren mit 8 bis 22 C-Atomen.

Geeignete Ölkomponenten sind weiterhin Dialkylcarbonsäureester, die lineare oder verzweigte Alkylketten mit 6 bis 22 C-Atomen tragen können.

Ebenso als Ölkomponente können Fettsäuretriglyceride verwendet werden, wobei unter diesen die natürlich vorkommenden Öle und Fette bevorzugt sind. So kommen beispielsweise natürliche, pflanzliche Öle, z. B. Olivenöl, Sonnenblurrienöl, Sojaöl, Erdnussöl, Rapsöl, Mandelöl, Palmöl aber auch die flüssigen Anteile des Kokosöls oder des Palmkernöls sowie tierische Öle wie z. B. Klauenöl, die flüssigen Anteile des Rindertalgs oder auch synthetische Triglyceride wie Umsetzungsprodukte mit Capryl-Caprinsäure-Gemischen oder mit Isostearinsäure, Triglyceride aus technischer Ölsäure oder aus Palmitinsäure-Ölsäure-Gemischen als Ölkomponenten in Frage.

Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan.

Weiterhin sind auch lineare oder verzweigte Fettalkohole wie Oleylalkohol oder Octyldodecanol, sowie Fettalkoholether wie Dicaprylyl-Ether einsetzbar.

Geeignete Siliconöle und -wachse sind z.B. Polydimethylsiloxane, Cyclomethylsiloxane, sowie nicht erfindungsgemäß beanspruchte aryl- oder alkyl- oder alkoxy-substituierte Polymethylsiloxane.

Weiterhin können zusätzlich Tenside, Emulgatoren oder Dispergierhilfsmittel verwendet werden. Dabei handelt es sich vorzugsweise um nichtionische, anionische, kationische oder amphotere Tenside bzw. Emulgatoren.

Als nichtionogene Emulgatoren oder Tenside kommen Verbindungen aus mindestens einer der folgenden Gruppen in Frage:

- Anlagerungsprodukte von 2 bis 30 mol Ethylenoxid und/oder 0 bis 5 mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe
- $C_{12/18}$-Fettsäuremono- und -diester von Anlagerungsprodukten von 1 bis 30 mol Ethylenoxid an Glycerin
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte
- Alkylmono- und -oligoglycoside mit 8 bis 22 Kohlenstoffatomen im Alkylrest und deren Ethylenoxidanlagerungsprodukte
- Anlagerungsprodukte von 2 bis 200 mol Ethylenoxid an Ricinusöl und/oder gehärtetes Ricinusöl
- Partialester auf Basis linearer, verzweigter, ungesättigter bzw. gesättigter $C_{6-22}$-Fettsäuren, Ricinolsäure sowie 12-Hydroxystearinsäure und Glycerin, Polyglycerin, Pentaerythrit, Dipentaerythrit, Zuckeralkohole (z. B. Sorbit), Alkylglucoside (z. B. Methylglucosid, Laurylglucosid oder Cetearylglucosid) sowie Polyglucoside (z. B. Cellulose). Dabei ist die Verwendung von Partialestern des Glycerins und des Polylglycerins bevorzugt. Dabei handelt es sich z.B. um Glycerinoleat, Glycerinisostearat, Polyglycerinlaurate, Polyglycerinisostearate, Polyglycerinoleate, Polyglycerinpolyricinoleate, Polyglycerin-Poly-12-hydroxystearate, Di-stearoyl Polyglyceryl-3 Dimer Dilinoleat oder Polyglyceryl-4 Diisostearate Polyhydroxystearate Sebacate.
- Mono-, Di- und Trialkylphosphate sowie Mono-, Di- und/oder Tri-PEG-alkylphosphate und deren Salze
- Polysiloxan-Polyether-Copolymere (Dimethicone Copolyole) die keinen erfindungsgemäßen Domänencharakter aufweisen, wie z.B. PEG/PPG-20/6 Dimethicone, PEG/PPG-20/20 Dimethicone, Bis-PEG/PPG-20/20 Dimethicone,

PEG-12 oder PEG-14 Dimethicone, PEG/PPG-14/4 oder 14/12 oder 20/20 oder 18/18 oder 17/18 oder 15/15. Besonders geeignet sind hierbei Produkte wie Bis-PEG/PPG-14/14 Dimethicone oder PEG/PPG-16/16 PEG/PPG-16/16 Dimethicone

- Polysiloxan-Polyalkyl-Polyether-Copolymere, die keinen erfindungsgemäßen Domänen-charakter aufweisen, bzw. entsprechende Derivate, wie z.B. Lauryl oder Cetyl Dimethicone Copolyole,
- Mischester aus Pentaerythrit, Fettsäuren, Citronensäure und Fettalkohol gemäß DE-B-1 165 574 und/oder Mischester von Fettsäuren mit 6 bis 22 Kohlenstoffatomen, Methylglucose und Polyolen, vorzugsweise Glycerin oder Polyglycerin.

Auch können anionische Emulgatoren oder Tenside zusätzlich verwendet werden.

Diese enthalten hydrophile anionische Gruppen wie z.B. Carboxylat-, Sulfat-, Sulfonat- oder Phosphat-Gruppen und einen lipophilen Rest. Hautverträgliche anionische Tenside sind dem Fachmann in großer Zahl bekannt und im Handel erhältlich. Dabei handelt es sich insbesondere um Alkylsulfate oder Alkylsphosphate in Form ihrer Alkali, Ammonium- oder Alkanolammoniumsalze, Alklyethersulfate, Alkylethercarboxylate, Acylsarkosinate sowie Sulfosuccinate und Acylglutamate in Form ihrer Alkali oder Ammoniumsalze. Als anionische Emulgatoren gelten auch neutralisierte oder teilneutralisierte Zitronensäureester, wie etwa Glyceryl Stearate Citrate, oder teilverseiftes Glyceryl Stearate (Glyceryl Stearate SE).

Auch kationische Emulgatoren und Tenside können zugesetzt werden.

Als solche sind insbesondere quartäre Ammoniumverbindungen verwendbar, etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid. Weiterhin können Monoalklyamidoquats wie z.B. Palmitamidopropyltrimethylammoniumchlorid oder entsprechende Dialkylamidoquats eingesetzt werden. Weiterhin können biologisch gut abbaubare quarternäre Esterverbindungen eingesetzt werden, bei denen es sich meist um quaternierte Fettsäureester auf Basis von Mono-, Di- oder Triethanolamin handelt. Weiterhin können Alkylguanidiniumsalze als kationische Emulgatoren hinzugefügt sein.

Weiterhin ist es möglich, amphotere Tenside wie z.B. Betaine, Amphoacetate oder Amphopropionate zusammen mit den erfindungsgemäßen Polyglycerinestern einzusetzen.

Weiterhin können bekannte Stabilisatoren und Verdicker für Öl- und Wasserphasen eingesetzt werden.

Zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Vorzugsweise sind diese anorganischen Ölphasenverdicker dabei hydrophob modifiziert.

Als Konsistenzgeber für Öl-in-Wasser-Emulsionen kommen in erster Linie Fettalkohole oder Hydroxyfettalkholole mit 12 bis 22 und vorzugsweise 16 bis 18 Kohlenstoffatomen und daneben Partialglyceride, Fettsäuren oder Hydroxyfettsäuren in Betracht.

Geeignete Verdickungsmittel für Wasserphasen sind beispielsweise Polysaccharide, insbesondere Xanthan-Gum, Guar und Guarderviate, Agar-Agar, Alginate und Tylosen, Cellulose und Cellulosederivate, wie z.B. Carboxymethylcellulose, Hydroxyethylcellulose, Hydroxymethlypropylcellulose, außerdem alkylmodifizierte Zuckerderivate wie z.B. Cetylhydroxyethylcellulose, ferner höhermolekulare Polyethylenglycolmono- und diester von Fettsäuren, Carbomere (vernetzte Polyacrylate), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside.

Unter Wirkstoffen sind beispielsweise Tocopherol, Tocopherolacetat, Tocopherolpalmitat, Ascorbinsäure, Desoxyribonucleinsäure, Coenzym Q10, Retinol- und Retinylderivate, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe zu verstehen.

Geeignete UV-Filter, Selbstbräuner, Konservierungsmittel, Antioxidantien, Hydrotrope, Antiperspirant-Wirkstoff, Deodorantien, Farbstoffe, Parfümöle, Insektrepellents wie sie im Stand der Technik beschrieben sind, können außerdem eingesetzt werden.

Ausführungsbeispiele:

Beispiel 1 (nicht erfindungsgemäß):

Herstellung eines domänenartig mit SiH-Funktionen durchsetzten Polydimethylsiloxan-Poly(methylwasserstoff)siloxan-Copolymeren

In einem 2-1-Vierhalsrundkolben mit KPG-Rührer, Rückflusskühler und Stickstoffüberleitung werden 16,3 g Hexamethyldisiloxan gemeinsam mit 256,6 g eines Poly(methylwasserstoff)siloxans (Molmasse: 2.868,1 g/mol, SiH-Wert: 15,69 Val/kg)) und 867,7 g Decamethylcyclopentasiloxan sowie mit 68,5 g eines vorgetrockneten Purolite C 150 MBH (vernetztes, makroporöses sulfonsaures Polystyrolharz) versetzt und unter Rühren wird die Reaktionsmischung für 6 Stunden auf 60 °C erwärmt. Der sulfonsaure Festphasenkatalysator wird nach Abkühlen der Reaktionsmatrix durch Filtration entfernt.

Beispiel 2 (erfindungsgemäß):

Herstellung eines domänenartig strukturierten Alkylpolyethersiloxans:

In einem 250-ml-Vierhalskolben mit KPG-Rührer, Rückflusskühler und Stickstoffüberleitung werden 100 g des domänenartig strukturierten Polydimethylsiloxan-Poly(methylwasserstoff)siloxan-Copolymeren (SiH-Wert: 3,53 Val/kg) auf 90 °C erhitzt und mit 10 ppm Platin (bezogen auf den Gesamtansatz in Form von cis-Di-amminoplatin(II)chlorid) versetzt. 30 g Hexadecen-1 werden hinzugegeben, wobei die Hydrosilylierungsreaktion einsetzt. Nach ca. 30 Minuten versetzt man die Reaktionsmischung mit 47,7 g eines aus Ethylenoxideinheiten aufgebauten, hydroxyfunktionellen Allylpolyethers, der ein mittleres Molekulargewicht von ca. 500 g/mol aufweist. Eine Stunde nach beendeter Reaktandzugabe werden weitere 33,3 g Hexadecen-1 hinzugegeben und man belässt den Ansatz für weitere 2 Stunden bei Reaktionstemperatur. Gasvolumetrische SiH-Bestimmung (Zersetzung einer aliquoten Probemenge mit Natriumbutylatlösung an einer Gasbürette) sichert einen quantitativen Umsatz. Das nahezu farblose, jedoch trübe Alkylpolyethersiloxan ist direkt als Emulgator einsetzbar.

Beispiel 3 (nicht erfindungsgemäß):

In Analogie zu Beispiel 2 werden 100 g eines statistisch gleichverteilten Polydimethylsiloxan-Poly(methylwasserstoff)-siloxan-Copolymeren (SiH-Wert: 3,53 Val/kg) auf 90 °C erhitzt und mit 10 ppm Platin (bezogen auf den Gesamtansatz in Form von cis-Diamminoplatin(II)chlorid) versetzt. Nach Zugabe von 30 g Hexadecen-1 lässt man den Ansatz eine halbe Stunde rühren und ergänzt die Reaktionsmischung durch die Zugabe von 47,7 g eines aus Ethylenoxideinheiten aufgebauten, hydroxyfunktionellen Allylpolyethers (mittleres Molekulargewicht von ca. 500 g/mol). Nach einer Stunde werden weitere 33,3 g Hexadecen-1 hinzugegeben und man lässt 2 Stunden nachreagieren. Gasvolumetrische SiH-Bestimmung (Zersetzung einer aliquoten Probemenge mit Natriumbutylatlösung an einer Gasbürette) belegt quantitativen Umsatz. Das nahezu farblose, klare Alkylpolyethersiloxan ist direkt als Emulgator einsetzbar.

Beispiel 4 (erfindungsgemäß) :

Herstellung eines domänenartig strukturierten Alkylsiloxans mit ammoniumfunktionellen Gruppen

In einem 250-ml-Vierhalskolben mit KPG-Rührer, Rückflusskühler und Stickstoffüberleitung werden 100 g des gemäß Beispiel 1 gewonnenen, domänenartig strukturierten Polydimethylsiloxan-Poly(methylwasserstoff)siloxan-Copolymeren (SiH-Wert: 3,53 Val/kg) auf 90 °C erhitzt und mit 10 ppm Platin (bezogen auf den Gesamtansatz in Form von cis-Diamminoplatin(II)chlorid) versetzt. 55,5 g Hexadecen-1 werden hinzugegeben, wobei die Hydrosilylierungsreaktion einsetzt. Nach ca. 30 Minuten gibt man 15,7 g Allylglycidylether hinzu. Man belässt den Ansatz 2 Stunden bei Reaktionstemperatur und führt dann eine gasvolumetrische SiH-Bestimmung (Zersetzung einer aliquoten Probemenge mit Natriumbutylatlösung an einer Gasbürette) durch, die einen quantitativen Umsatz bestätigt. Man ersetzt den Rückflusskühler durch eine Destillationsbrücke und destilliert bei vermindertem Druck (30 mPas) überschüssigen Allylglycidylether neben einer kleinen Menge an Siloxancyclen ab. Der Epoxysauerstoffgehalt beträgt im erkalteten Reaktionsansatz 1 %.

Im nächsten Schritt werden 167,6 g des Epoxy-modifizierten Alkylsiloxans bei 25 °C in einem 500-ml-Vierhalskolben zu einer Mischung bestehend aus 31,6 g Cocosfettsäureamidamin (Amid CNF/Degussa) und 6,6 g Essigsäure sowie 50 g Isopropanol unter Rühren zugetropft. Man erhitzt für 6 Stunden auf Rückflusstemperatur und lässt dann den klaren Kolbeninhalt erkalten.

Anwendungsbeispiele:

Die nachfolgenden Anwendungsbeispiele sollen den Gegenstand der Erfindung näher erläutern, ohne ihn auf diese Beispiele einzuschränken. Die Konzentrationsangaben in allen Beispielen sind als Gew.-% angegeben.

Anwendungsbeispiel 1:

Dieses Anwendungsbeispiel soll zeigen, dass ein typisches erfindungsgemäß aufgebautes, domänenartig strukturiertes, kammartiges Alklpolyethersiloxan (Synthesebeispiel 1) als Wasser-in-öl-Emulgator hervorragende Anwendungseigenschaften zeigt, die gegenüber den bereits exzellenten Anwendungseigenschaften von Wasser-in-Öl-Emulgatoren auf Basis statistisch aufgebauter, kammartiger Alkylpolyethersiloxane zusätzliche Vorteile aufweisen.

| | Emulsionen | 1 | V1 | 2 | V2 |
|---|---|---|---|---|---|
| A | Domänenartig aufgebautes Alkyl-Polyethersiloxan aus Synthesebeispiel 2 | 2,00 % | | 2,00 % | |
| | Statistisch aufgebautes Alkyl-Polyethersiloxan aus Vgl.Bsp.3 | | 2,00 % | | 2,00 % |
| | Hydrogenated Castor Oil | 0,10 % | 0,10 % | | 0,10 % |
| | Microcrystalline Wax | 0,10 % | 0,10 % | | 0,10 % |
| | Caprylic Capric Triglyceride | 8,90 % | 8,90 % | | |
| | Ethylhexyl Palmitate | 8,90 % | 8,90 % | 6,00 % | 6,00 % |
| | Paraffinum Perliquidum | | | 11,80 % | 11,80 % |
| B | Glycerin | 3,10 % | 3,10 % | 3,10 % | 3,10 % |
| | NaCl | 0,80 % | 0,80 % | 0,80 % | 0,80 % |
| | Bronopol | 0,05 % | 0,05 % | 0,05 % | 0,05 % |
| | Wasser | ad 100 | ad 100 | ad 100 | ad 100 |
| | Stabilität | stabil | stabil | stabil | stabil |
| | Viskosität* [Pas] | 36 | 45 | 31 | 37 |
| | Applikationseigenschaften | sehr gut | gut | sehr gut | gut |
| *Brookfield RVT Spindel C, 10 rpm | | | | | |

Die erfindungsgemäßen Wasser-in-Öl-Emulsionen 1 und 2 zeigten dabei absolut vergleichbare Stabilitäten wie sie von statistisch modifizierten Alkylpolyethersiloxanen bekannt sind. Darüber hinaus konnte in den erfindungsgemäßen Beispielemulsionen 1 und 2 jedoch eine niedrigere Emulsionsviskosität beobachtet werden.

Eine niedrigere Emulsionsviskosität bei gleich bleibender Stabilität ist im Fall von W/O insofern vorteilhaft, als sie eine bessere Verteilbarkeit der Emulsion auf der Haut ermöglicht.

Zusätzlich zeigten die erfindungsgemäßen Emulsionen ein generell etwas leichteres Hautgefühl als dies mit den Vergleichsemulsionen auf Basis statistisch modifizierter Alkylpolyethersiloxane der Fall war.

Somit zeichnen sich die erfindungsgemäßen blockartigen Alkylpolyethersiloxane durch überraschend positive Applikationseigenschaften aus.

Anwendungsbeispiel 2:

Für die Formulierung von Wasser-in-Silicon-Emulsionen, wie sie etwa in den Bereichen Antiperspirantien/ Deodorants oder im Bereich der dekorativen Kosmetik überwiegend Anwendung finden, werden in der Praxis bisher meist Polyethersiloxane mit einer linear aufgebauten $\alpha$-$\omega$-Struktur verwendet. Grund hierfür ist die ausgezeichnete Kompatibilität des unmodifizierten Siloxanrückens eines solchen Moleküls mit der Siliconölphase.

Wenngleich sich solche Emulgatoren durch ein sehr angenehmes, samtig-seidiges Hautgefühl auszeichnen ist ihr Stabilisierungspotential doch begrenzt.

Erfindungsgemäße domänenartig strukturierte, kammartige Alkylpolyethersiloxane wie aus Synthesebeispiel 1 führen nun insbesondere im Bereich der W/Si-Emulsionen zu einem ähnlich angenehmen, samtig-seidigen Hautgefühl,

zeigen dabei aber eine deutlich verbesserte Stabilität (wie aus der Vergleichstabelle ersichtlich).

Als Vergleichsbeispiel wurde ein üblicher W/Si-Emulgator mit α-ω-Modifikation ausgewählt (Bis-PEG/PPG-14/14 Dimethicone).

| | Emulsionen | 1 | V1 | 2 | V2 |
|---|---|---|---|---|---|
| A | Domänenartig aufgebautes Alkyl-Polyethersiloxan aus Synthesebeispiel 2 | 2,00 % | | 1,00 % | |
| | Bis-PEG/PPG-14/14 Dimethicone | | 2,00 % | | 1,00 % |
| | Cyclopentasiloxane | 23,00 % | 23,00 % | 24,00 % | 24,00 % |
| B | NaCl | 0,60 % | 0,60 % | 0,60 % | 0,60 % |
| | Bronopol | 0,05 % | 0,05 % | 0,05 % | 0,05 % |
| | Wasser | ad 100 | ad 100 | ad 100 | ad 100 |
| | Stabilität | stabil | mäßig[1)] | stabil | schlecht[2)] |
| | Viskosität* [Pas] | 23 | 26 | 20 | 31 |
| | Applikationseigenschaften | sehr gut | sehr gut | sehr gut | sehr gut |

* Brookfield RVT Spindel C, 10 rpm
[1)] deutliche Wasserseparation nach drei Gefrier-Tau-Zyklen (3 x -15 °C/20 °C)
[2)] Emulsionszersetzung nach drei-Tau-Zyklen (3 x -15 °C/20 °C); deutliche Wasserseparation nach einem Monat Lagerung bei 45 °C

Bei den untersuchten Emulsionen handelt es sich um ausgesprochen kritische, wachsfreie Rezepturen. Insbesondere die Emulsionen mit einem reduzierten Emulgatorgehalt von 1 % offenbaren die enorm starke Stabilisierungswirkung der erfindungsgemäßen Polyethersiloxane.

Anwendungsbeispiel 3:

Dieses Anwendungsbeispiel soll zeigen, dass ein typisches erfindungsgemäß aufgebautes, domänenartig strukturiertes, kammartiges Alkylpolyethersiloxan (Synthesebeispiel 2) als Inhaltsstoff in Hautreingungsmitteln hervorragende Anwendungseigenschaften zeigt, das gegenüber den bereits exzellenten Anwendungseigenschaften bekannter Rückfettungsmittel auf Basis statistisch aufgebauter, kammartiger Alkylpolyethersiloxane zusätzliche Vorteile aufweist.

| Hautreingungsmittel | 1 | V1 | V2 | V3 |
|---|---|---|---|---|
| Sodium Laureth Sulfate, 28 % | 32,0 % | 32,0 % | 32,0 % | 32,0 % |
| Parfüm | 0,5 % | 0,5 % | 0,5 % | 0,5 % |
| Domänenartig aufgebautes Alkyl-Polyethersiloxan aus Synthesebeispiel 2 | 0,5 % | - | - | - |
| Statistisch aufgebautes Alkyl-Polyethersiloxan aus Vgl.Bsp.3 ABIL® EM 90 Cetyl PEG/PPG-10/1 Dimethicone | - | 0,5 % | - | - |
| ABIL® B 88184 PEG/PPG-20/6 Dimethicone | - | - | 0,5 % | - |
| ABIL® B 8832 Bis-PEG/PPG-20/20 Dimethicone | - | - | - | 0,5 % |
| REWOTERIC® AM C Sodium Cocoamphoacetate 30 % | 6,0 % | 6,0 % | 6,0 % | 6,0 % |
| TEGO Betain F 50 | 6,0 % | 6,0 % | 6,0 % | 6,0 % |
| Citric Acid (30 % in water) | 1,5 % | 1,5 % | 1,5 % | 1,5 % |
| ANTIL® 200 PEG-200 Hydrogenated Glyceryl Palmate; PEG-7 Glyceryl Cocoate | 1,5 % | 1,5 % | 1,5 % | 1,5 % |
| Sodium Chloride | 1 % | 1 % | 1 % | 1 % |
| Wasser | ad 100 % | ad 100 % | ad 100 % | ad 100 % |

(fortgesetzt)

| | | | | |
|---|---|---|---|---|
| Bewertung: | | | | |
| Hautgefühl | 5 | 4,2 | 3,2 | 4,5 |

Das erfindungsgemäße Hautreinigungsmittel 1 zeigt im Vergleich mit den Vergleichsprodukten V1 bis V3 das beste Hautgefühl. Dieser Parameter bewertet Hautglätte und Weichheit, die Abwesenheit klebriger Rückstände und die empfundene Hautfeuchtigkeit im in-vivo-Versuch auf menschlicher Haut. Auch die Formulierung V3 in der mit Bis-PEG/PPG-20/20 Dimethicone ein Polyethersiloxan mit einer ungestörten Polydimethylsiloxane verwendet wurde, zeigt eine schwächere Bewertung im Hautgefühl als das erfindungsgemäße Hautreinigungsmittel 1. Die in der Tabelle aufgeführte Bewertung erfolgte nach der Skala: 1 = schlecht bis 5 = hervorragend.

Anwendungsbeispiel 4:

Dieses Anwendungsbeispiel soll zeigen, dass ein typisches erfindungsgemäß aufgebautes, domänenartig strukturiertes, kammartiges Alkylsiloxan mit ammoniumfunktionellen Gruppen (Synthesebeispiel 4) als Konditioniermittel für Haarpflegeprodukte hervorragende Anwendungseigenschaften zeigt, die gegenüber den bereits exzellenten Anwendungseigenschaften bekannter Konditioniermittel auf Basis modifizierter Siloxane zusätzliche Vorteile aufweisen.

| Haarshampoos | 1 | V1 | 2 | V2 |
|---|---|---|---|---|
| Sodium Laureth Sulfate, 28 % | 32,0 % | 32,0 % | 32,0 % | 32,0 % |
| Parfüm | 0,5 % | 0,5 % | 0,5 % | 0,5 % |
| Domänenartig aufgebautes Alkylsiloxan mit Ammoniumfunktionellen Gruppen aus Synthesebeispiel 4 | 0,5 % | - | 0,5 % | - |
| ABIL® Quat 3272 Quaternium-80 | - | 0,5 % | - | 0,5 % |
| Jaguar C 162 Hydroxypropyl Guar Hydroxypropyltrimonium Chloride | - | - | 0,2 % | 0,2 % |
| REWOTERIC® AM C Sodium Cocoamphoacetate 30 % | 6,0 % | 6,0 % | 6,0 % | 6,0 % |
| TEGO Betain F 50 | 6,0 % | 6,0 % | 6,0 % | 6,0 % |
| Citric Acid (30 % in water) | 1,5 % | 1,5 % | 1,5 % | 1,5 % |
| ANTIL® 200 PEG-200 Hydrogenated Glyceryl Palmate; PEG-7 Glyceryl Cocoate | 1,5 % | 1,5 % | 1,5 % | 1,5 % |
| Sodium Chloride | 1 % | 1 % | 1 % | 1 % |
| Wasser | ad 100 % | ad 100 % | ad 100 % | ad 100 % |
| Bewertung: | | | | |
| Nasskämmbarkeit | 3,5 | 2,5 | 4,5 | 3,0 |
| Nassgriff | 4,2 | 2,5 | 4,5 | 3,2 |
| Trockenkämmbarkeit | 4,0 | 3,5 | 4,5 | 3,5 |
| Trockengriff | 4,5 | 3,2 | 5,0 | 3,8 |

Die erfindungsgemäßen Haarshampoos 1 und 2 zeigen im Vergleich mit den Vergleichsprodukten V1 und V2 jeweils bessere konditionierende Eigenschaften. Insbesondere die Bewertung des Griffs der nassen Haare sowie nach dem Trocknen fällt bei den Shampoos 1 und 2 mit dem erfindungsgemäßen Konditioniermittel nach Beispiel 4 signifikant besser aus. Die in der Tabelle aufgeführten Bewertungen erfolgen nach der Skala: 1 = schlecht bis 5 = hervorragend. Die Bewertung erfolgt anhand von Haarsträhnen durch ein Bewertungspanel.

## Patentansprüche

**1.** Verfahren zur Herstellung von organomodifizierten Siloxanen mit domänenartiger Verteilung erhalten durch teilweise oder vollständige Umsetzung von

A) Wasserstoffsiloxanen mit einem Verteilungsgrad (persistency ratio) ($\eta$) der Komponenten [A] und [B] im Copolymeren [AB]

$$\eta = \frac{[A]\,[B]}{[AB]}$$

von $\eta > 1$, vorzugsweise > 1,1, insbesondere $\geq 1{,}2$, mit
B) olefinisch und/oder acetylenisch ungesättigten Verbindungen.

**2.** Verfahren zur Herstellung von organomodifizierten Siloxanen gemäß Anspruch 1, **dadurch** gekennzeichet, dass man zur Herstellung der Methylsiloxane ein Gemisch aus Hexamethyldisiloxan, Poly(methyl)wasserstoffsiloxan und Siloxancyclen als Ausgangsmaterial äquilibriert.

**3.** Verfahren zur Herstellung von organomodifizierten Siloxanen gemäß einem der Ansprüche 1 oder 2, **dadurch gekennzeichnet, dass** olefinisch ungesättigte Verbindungen der allgemeinen Formel (1) $CH_2=CHR-(CH_2)_n-A$ verwendet werden, worin A mindestens ein Rest ist, ausgesucht aus der Gruppe der

1. gegebenenfalls substituierten, gegebenenfalls funktionelle Gruppen enthaltenden linearen und/oder cyclischen Kohlenwasserstoffe,
2. statistisch oder blockweise aufgebauten Oxyalkylenketten $-(OAlk)_a-R$,
worin

R H, ein gegebenenfalls substituierter und/oder gegebenenfalls Heteroatome enthaltender $C_{1-18}$-Alkylrest oder der Rest einer einbasischen und/oder mehrbasischen $C_{1-22}$-Carbonsäure welche gegebenenfalls Hydroxylgruppen enthalten kann, der Rest einer anorganischen Säure ausgesucht aus der Gruppe Schwefelsäure, Sulfonsäure Phosphorsäure,
Oalk der Rest $-(EO)_b-(PO)_c-(BO)_d-(DO)_e-(SO)_f-$ mit

EO Ethylenoxidrest,
PO Propylenoxidrest,
BO Butylenoxydrest,
DO Dodecenyloxidrest,
SO Styroloxidrest,
b,c,d,e,f $\geq 0$ sind, insbesondere
b 0 bis 50,
c 0 bis 50,
d,e,f gleich oder verschieden 0 bis 10 und die Summe
b+c+d+e+f a beträgt und
a 1 bis 50, vorzugsweise 1 bis 30, insbesondere 3 bis 20 ist,

und

3. Oxirangruppen enthaltende Reste der Formeln 3a bis 3d

3a 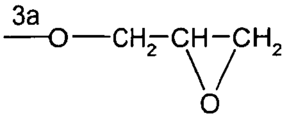 3b 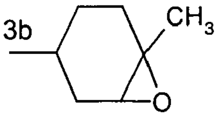

(und bei x = 0)

3c 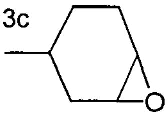 3d 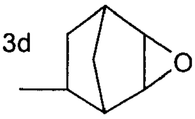

4. mindestens ein Amin-/Ammoniumrest, -MZ, $-[M-Z]^{g+}$ $h^{*}X^{j-}$, worin

M ein zweiwertiger Rest ist, ausgewählt aus der Gruppe

4a 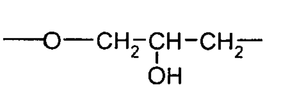 4b 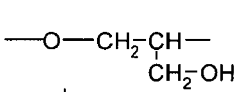 4c 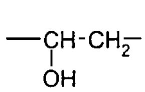 4d 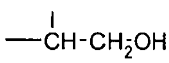

4e 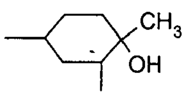 4f 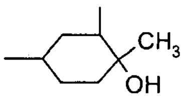 4g 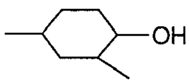 4h 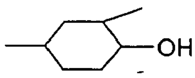

4j 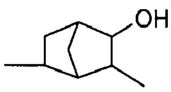 4k 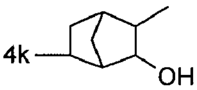

und Z einer der Reste

4m 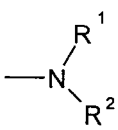 4n 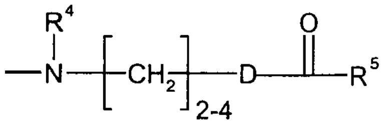 4o 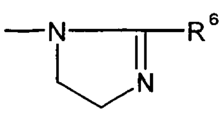

4p 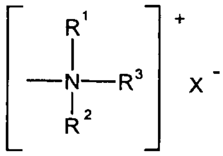 4q 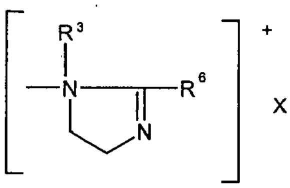 4r 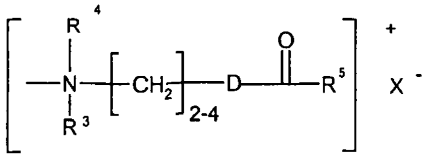

sein kann, worin

$R^1$, $R^2$, $R^3$, $R^4$ unabhängig voneinander H, $C_{1-22}$-Alkylreste die auch Hydroxylgruppen enthalten können,

$R^5$, $R^6$ $C_{1-22}$-Alkylreste die auch Hydroxylgruppen enthalten können,
D -O- oder -$NR^7$- mit $R^7$ = Alkyl- oder Hydroxyalkylrest mit 1-4 C-Atomen,
$X^-$ ein organisches oder anorganisches Anion sein kann,
h*j gleich dem Zahlenwert von g ist,

5. Polyhydroxyorganylreste der allgemeinen Formel -$R^8$-PH, wobei der Rest

$R^8$ als Spacer zwischen Siloxangerüst und Polyhydroxyorganylrest bzw. Zuckerrest fungiert und von aus dem Stand der Technik für polyhydroxyorganyl- bzw. zuckermodifizierte Siloxane bekannter Art ist,
PH ein Polyhydroxyorganylrest ist, der eine definierte Anzahl n von (C-OH)-Gruppen enthält, wobei $n \geq 2$ ist, bevorzugt 5 bis 15,

aus der Gruppe Mono-, Di-, Oligo- oder Polysaccharid, deren Glycoside oder entsprechende Derivate insbesondere Glucose, Maltose, Raffinose, Sorbit, Glucosamin, Glucopyranosylamin, Glucamin, N-Methylglucamin, Isomaltamin, Gluconsäure, Heptagluconsäure;

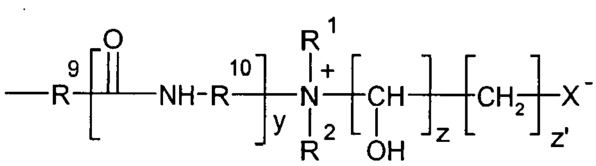

6. Betaingruppen,
worin

X -COO-, $SO_3^-$ , $PO_4^{2-}$,
$R^9$ Alkylenreste mit bis zu 10 C-Atomen,
$R^{10}$ Alkylenreste mit bis zu 2 bis 6 C-Atomen sind,
y 0 oder 1,
Z 0 oder 1,
z' 1,2 oder 3,

und

$R^1$, $R^2$ gleich oder verschieden die oben genannte Bedeutung haben, zusammen einen Imidazolinring bilden oder $-CH_2-CH_2-OH$, $-(CH_2)_z-X^-$, sind,

7. Guanidingruppen der Formeln (6a, 6b oder 6c)

$R^{11}$ = -N-G (6a)

$R^{11}$ = $-N-Q^+ A^-$ (6b)

$R^{11}$ = - $(N)_x$-S (6c)

worin

G eine Guanidinogruppe mit der allgemeinen Formel ($6a^1$,$6a^2$)

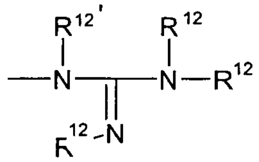

(6a¹)

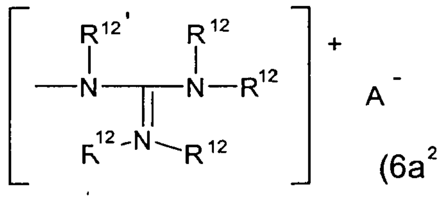

(6a²)

und/oder deren Salze oder Hydrate ist, in denen

$R^{12}$ unabhängig voneinander, Wasserstoff oder ein gegebenenfalls verzweigter, gegebenenfalls Doppelbindungen enthaltender Kohlenwasserstoffrest, oder
$R^{12'}$ $R^{12}$ sein kann oder eine Alkylengruppe, die mit M über Kohlenstoff- oder Heteroatome verknüpft ist und so einen 5 bis 8-gliedrigen Ring bildet und
N ein zwei- oder mehrwertiger Kohlenwasserstoffrest mit mindestens 4 Kohlenstoffatomen ist, der eine Hydroxylgruppe aufweist und der durch ein oder mehrere Sauerstoffatome oder Stickstoffatome oder quaternäre Ammoniumgruppen oder Ester oder Amidfunktionen unterbrochen sein kann,
$Q^+$ ein Rest der Formel (6d)

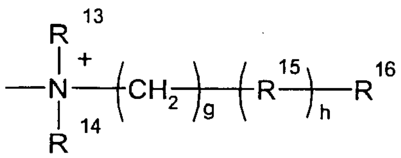

(6d)

ist,

$R^{13}$, $R^{14}$ Alkylreste mit 1 bis 4 Kohlenstoffatomen sind,
$R^{15}$

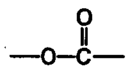 oder 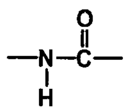

ist,
$R^{16}$ ein einwertiger Kohlenwasserstoffrest mit 1 bis 22 Kohlenstoffatomen sein kann,
g 0 bis 6
h 0 oder 1,

$A^-$ ein anorganisches oder organisches Anion, das von einer üblichen physiologisch verträglichen Säure HA herrührt,
S H, ein Polyalkylenoxid-Polyether der allgemeinen Formel $C_mH_{2m}O(C_2H_4O)_n(C_3H_6O)_oR^{17}$
worin

m 1 bis 6, insbesondere 3, 6,
n,o unabhängig voneinander 0 bis 100, insbesondere 0 bis 20 und der Polyether ein Molekulargewicht zwischen 100 und 6.000 g/mol aufweist und
$R^{17}$ H oder ein gegebenenfalls verzweigter, gegebenenfalls Doppelbindungen enthaltender aromatischer oder alicyclischer Kohlenwasserstoffrest mit 2 bis 30 C-Atomen, vorzugsweise 4 bis 22 C-Atomen, oder eine UV-absorbierende Gruppe, insbesondere Zimtsäure oder Methoxyzimtsäure, ist.

**4.** Organomodifizierte Siloxane mit Blockcharakter hergestellt gemäß mindestens einem der Ansprüche 1 bis 3.

**5.** Verwendung der organomodifizierten Siloxane mit Blockcharakter gemäß Anspruch 4 zur Herstellung und Stabilisierung kosmetischer Formulierungen zur Reinigung und Pflege von Haut und Haar, in Sonnenschutzprodukten, in Antitranspirantien/Deodorantien, sowie in pigmenthaltigen Formulierungen aus dem Bereich der dekorativen Kosmetik.

**6.** Kosmetische oder pharmazeutische Formulierungen die ein oder mehrere organomodifizierte Siloxane mit Blockcharakter gemäß Anspruch 4 enthalten.

**7.** Verwendung der organomodifizierten Siloxane mit Blockcharakter gemäß Anspruch 4 in Mitteln zur Reinigung und Pflege harter Oberflächen, sowie zur Reinigung und Pflege von Textilien.

**8.** Verwendung der organomodifizierten Siloxane mit Blockcharakter gemäß Anspruch 4 als Additive in Farben, Lacken, Überzügen und Beschichtungen.

Europäisches Patentamt

# EUROPÄISCHER TEILRECHERCHENBERICHT

der nach Regel 45 des Europäischen Patent-übereinkommens für das weitere Verfahren als europäischer Recherchenbericht gilt

Nummer der Anmeldung
EP 06 02 3972

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X<br><br><br><br><br><br><br><br><br><br><br>Y | CANCOUET P ET AL: "FUNCTIONAL POLYSILOXANES. II. NEIGHBORING EFFECT IN THE HYDROSILYLATION OF POLY(HYDROGENMETHYLSILOXANE-CO-DIMETHYLSILOXANE)S BY ALLYGLYCIDYLETHER" JOURNAL OF POLYMER SCIENCE, POLYMER CHEMISTRY EDITION, INTERSCIENCE POUBLISHERS, NEW YORK, NY, US, Bd. 38, Nr. 5, 1. März 2000 (2000-03-01), Seiten 837-845, XP000893976<br>ISSN: 0360-6376<br>* Zusammenfassung *<br>* Seite 837, rechte Spalte, letzter Absatz - Seite 238, linke Spalte, Absatz 1 *<br>* Tabelle 1 *<br>* Seite 838, rechte Spalte, Synthesis, Absatz 1 *<br>* Seite 844, Conclusion *<br>-----<br>-/-- | 1-4<br><br><br><br><br><br><br><br><br><br><br>5-8 | INV.<br>A61K8/894<br>A61K8/898<br>A61Q1/02<br>A61Q15/00<br>A61Q17/04<br>A61Q19/10<br>C08G77/10<br>C08G77/12<br>C08G77/14<br>C08G77/26<br>C08G77/38<br>C08G77/388<br>C08G77/46<br>C09D7/12<br>C11D1/82<br>C11D3/37 |
| | | | RECHERCHIERTE SACHGEBIETE (IPC) |
| | | | A61K<br>A61Q<br>C08G<br>C09D<br>C11D |

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

1

| Recherchenort | Abschlußdatum der Recherche | Prüfer |
|---|---|---|
| München | 5. April 2007 | Hein, Friedrich |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

**EUROPÄISCHER TEILRECHERCHENBERICHT**

Nummer der Anmeldung
EP 06 02 3972

| EINSCHLÄGIGE DOKUMENTE | | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| T | CANCOUET P ET AL: "FUNCTIONAL POLYSILOXANES. I. MICROSTRUCTURE OF POLY(HYDROGENMETHYLSILOXANE-CO-DIMETHYLSILOXANE)S OBTAINED BY CATIONIC COPOLYMERIZATION" JOURNAL OF POLYMER SCIENCE, POLYMER CHEMISTRY EDITION, INTERSCIENCE POUBLISHERS, NEW YORK, NY, US, Bd. 38, Nr. 5, 1. März 2000 (2000-03-01), Seiten 826-836, XP000893975 ISSN: 0360-6376 * Zusammenfassung * * Seite 827, Materials * * Seite 832 * * Tabelle IV * ----- | 1,2 | |
| | | | RECHERCHIERTE SACHGEBIETE (IPC) |
| P,A | DE 10 2005 001039 A1 (GOLDSCHMIDT GMBH [DE]) 20. Juli 2006 (2006-07-20) * Absätze [0015] - [0021], [0023], [0040] - [0045] * * Ansprüche * ----- | 1,2,8 | |
| A | EP 1 439 200 A (GOLDSCHMIDT AG TH [DE]) 21. Juli 2004 (2004-07-21) * Ansprüche; Beispiel 1 * ----- | 1-8 | |
| A | DE 21 52 270 A1 (UNION CARBIDE CORP) 27. April 1972 (1972-04-27) * Ansprüche; Beispiele 7-10 * ----- | 1-8 | |
| Y | EP 0 125 779 A2 (DOW CORNING [US]) 21. November 1984 (1984-11-21) * Zusammenfassung * * Ansprüche; Beispiel 3 * ----- | 4-6 | |
| Y | JP 2002 167437 A (LION CORP; LION AKZO KK) 11. Juni 2002 (2002-06-11) * Zusammenfassung * ----- | 4-7 | |
| | -/-- | | |

1

EPO FORM 1503 03.82 (P04C12)

Europäisches Patentamt

# EUROPÄISCHER TEILRECHERCHENBERICHT

Nummer der Anmeldung
EP 06 02 3972

| EINSCHLÄGIGE DOKUMENTE | | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| Y | EP 1 288 246 A1 (WACKER CHEMIE GMBH [DE]) 5. März 2003 (2003-03-05) * Absatz [0068]; Ansprüche * ----- | 4-7 | |
| Y | EP 1 382 633 A (GOLDSCHMIDT AG TH [DE]) 21. Januar 2004 (2004-01-21) * Ansprüche * ----- | 4,8 | |
| | | | RECHERCHIERTE SACHGEBIETE (IPC) |

1

EPO FORM 1503 03.82 (P04C12)

Europäisches Patentamt

**UNVOLLSTÄNDIGE RECHERCHE ERGÄNZUNGSBLATT C**

Nummer der Anmeldung
EP 06 02 3972

Unvollständig recherchierte Ansprüche:
1-8

Grund für die Beschränkung der Recherche:

Der vorliegende Anspruch 1 bezieht sich auf organomodifizierte Siloxane, charakterisiert durch eine bestimmte gewünschte Eigenschaft, nämlich eine "domänenartige Verteilung". Was unter "domänenartiger Verteilung" bzw. "Verteilungsgrad (persistency ratio)" zu verstehen ist, geht aus Anspruch 1 nicht hervor, so dass der Anspruch das Erfordernis der Klarheit nicht erfüllt (Art. 84 EPÜ). Die Beschreibung gibt keine Stützung (Art. 84) und Offenbarung (Art. 83 EPÜ), wie derartige organomodifizierte Siloxane mit "domänenartiger Verteilung" anders erhalten werden können als durch die in Beispiel 1 sowie in Journal of Polymer Science, Part A: Polymer Chemistry, Vol. 38, 826-836 (2000) angegebene Methode. Die Verletzung der einschlägigen Erfordernisse ist so schwerwiegend, dass eine sinnvolle Recherche des gesamten beanspruchten Gegenstandes nicht durchgeführt werden konnte (Regel 45 EPÜ und Richtlinien B-VIII, 3).
Die Recherche von Ansprüchen 1-8 wurde deshalb auf die in Anspruch 2 sowie in Beispiel 1 und in Journal of Polymer Science, Part A: Polymer Chemistry, Vol. 38, 826-836 (2000) deutlich offenbarten Edukte, Methode und spezielle Katalysatoren beschränkt.

**ANHANG ZUM EUROPÄISCHEN RECHERCHENBERICHT ÜBER DIE EUROPÄISCHE PATENTANMELDUNG NR.** EP 06 02 3972

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

05-04-2007

| Im Recherchenbericht angeführtes Patentdokument | Datum der Veröffentlichung | Mitglied(er) der Patentfamilie | Datum der Veröffentlichung |
|---|---|---|---|
| DE 102005001039 A1 | 20-07-2006 | US 2006155089 A1 | 13-07-2006 |
| EP 1439200 A | 21-07-2004 | CA 2450173 A1 | 16-07-2004 |
| | | DE 10301355 A1 | 29-07-2004 |
| | | US 2006241270 A1 | 26-10-2006 |
| | | US 2004147703 A1 | 29-07-2004 |
| DE 2152270 A1 | 27-04-1972 | AU 464094 B2 | 14-08-1975 |
| | | AU 3452271 A | 27-09-1973 |
| | | BE 774193 A1 | 20-04-1972 |
| | | CA 970095 A1 | 24-06-1975 |
| | | FR 2111629 A5 | 02-06-1972 |
| | | GB 1366103 A | 11-09-1974 |
| | | JP 50010849 B | 24-04-1975 |
| | | NL 7114412 A | 25-04-1972 |
| | | US 3694405 A | 26-09-1972 |
| EP 0125779 A2 | 21-11-1984 | CA 1234575 A1 | 29-03-1988 |
| | | DE 3480933 D1 | 08-02-1990 |
| | | JP 1489590 C | 23-03-1989 |
| | | JP 59197432 A | 09-11-1984 |
| | | JP 63036620 B | 21-07-1988 |
| | | US 4532132 A | 30-07-1985 |
| JP 2002167437 A | 11-06-2002 | KEINE | |
| EP 1288246 A1 | 05-03-2003 | BR 0203112 A | 27-05-2003 |
| | | CN 1401683 A | 12-03-2003 |
| | | DE 10139963 A1 | 06-03-2003 |
| | | JP 2003073477 A | 12-03-2003 |
| | | US 2003045666 A1 | 06-03-2003 |
| EP 1382633 A | 21-01-2004 | CA 2433002 A1 | 19-01-2004 |
| | | DE 10232908 A1 | 29-01-2004 |
| | | US 2005257717 A1 | 24-11-2005 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0176884 A
- EP 1125574 A
- EP 0294642 B
- US 3234252 A
- US 4047958 A
- US 3427271 A
- US 6645842 B
- US 4698178 A
- US 5204433 A
- US 4891166 A
- US 4833225 A
- US 4609750 A
- DE 1165574 B


### In der Beschreibung aufgeführte Nicht-Patentliteratur


- **P. CANCOUET et al.** Functional Polysiloxanes, I. Microstructure of Poly(hydrogenmethylsiloxane-co-dimethylsiloxane)s Obtained by Cationic Copolymerization. *J. of Polymer Science, Par A: Polymer Chemistry,* 2000, vol. 38, 826-836